# EUROPEAN PATENT SPECIFICATION

(11) **EP 0 695 804 B1**
(45) Date of publication and mention of the grant of the patent: **18.08.2004**
(21) Application number: 95303933.6
(22) Date of filing: 08.06.1995
(51) Int. Cl.: C12N 15/61, C12N 9/90, C12N 1/20, C12P 19/24

(54) **DNA encoding for a recombinant enzyme for converting maltose into trehalose**
DNS, die für ein rekombinantes Enzym zur Umwandlung von Maltose in Trehalose kodiert
ADN codant pour une enzyme recombinante pour la conversion de maltose en trehalose

(30) Priority: 16.06.1994 JP 15639994
(43) Date of publication of application: 07.02.1996
(73) Proprietor: KABUSHIKI KAISHA HAYASHIBARA SEIBUTSU KAGAKU KENKYUJO, Okayama-shi Okayama (JP)
(72) Inventor: Kubota, Michio, Okayama-shi, Okayama (JP); Tsusaki, Keiji, Kurashiki-shi, Okayama (JP); Sugimoto, Toshiyuki, Okayama-shi, Okayama (JP)
(74) Representative: Daniels, Jeffrey Nicholas

(56) References cited:
- EP-A- 0 636 693
- EP-A- 0 671 470
- EP-A- 0 690 130
- EP-A- 0 693 558
- BIOSCIENCE BIOTECHNOLOGY AND BIOCHEMISTRY, vol. 59, no. 11, 1995, pages 2189-2190, XP000577071 NISHIMOTO ET AL.: "Existence of a novel enzyme converting Maltose into Trehalose"
- BIOSCIENCE BIOTECHNOLOGY AND BIOCHEMISTRY, vol. 60, no. 4, 1996, pages 640-644, XP002020456 NISHIMOTO ET AL.: "Purification and properties of a novel enzyme, Trehalose Synthase, from Pimelobacter sp. R48"
- BIOCHIMICA ET BIOPHYSICA ACTA, vol. 1290, 1996, pages 1-3, XP000612035 TSUSAKI ET AL.: "Cloning and sequencing of Trehalose Synthase gene from Pimelobacter sp. R48"
- BIOSCIENCE BIOTECHNOLOGY AND BIOCHEMISTRY, vol. 60, no. 5, 1996, pages 835-839, XP002020458 NISHIMOTO ET AL.: "Purification and characterization of a thermostable Trehalose Synthase from Thermus aquaticus"

## Description

The present invention relates to a DNA encoding a recombinant enzyme which converts maltose into trehalose, as well as to a replicable recombinant DNA, transformant, process for preparing the recombinant enzyme, and enzymatic conversion method of maltose.

Trehalose is a disaccharide which consists of 2 glucose molecules linked together with their reducing groups, and, naturally, it is present in bacteria, fungi, algae, insects, etc., in an extremely-small quantity. Having no reducing residue within the molecule, trehalose does not cause an unsatisfactory browning reaction even when heated in the presence of amino acids or the like, and because of this it can advantageously sweeten food products without fear of causing unsatisfactory coloration and deterioration. Trehalose, however, is far from being readily prepared in a desired amount by conventional methods, and, actually, it has not been scarcely used for sweetening food products.

Conventional methods are roughly classified into 2 groups, i.e. the one using cells of microorganisms and the other employing a multi-enzymatic system wherein enzymes are allowed to act on saccharides. The former, as disclosed in JP-A-154,485/75, is a method which comprises allowing to grow microorganisms such as bacteria and yeasts in a nutrient culture medium, and collecting trehalose from the resultant culture. The latter, as disclosed in JP-A-216,695/83, is a method which comprises providing maltose as a substrate, allowing a multi-enzymatic system using maltose- and trehalose-phosphorylases to act on maltose, and isolating the formed trehalose from the reaction system. Although the former facilitates the growth of microorganisms without special difficulty, it has a drawback that the resultant culture contains at most 15 w/w % trehalose, on a dry solid basis (d.s.b.). While the latter enables the separation of trehalose with a relative easiness, but it is theoretically difficult to increase the trehalose yield by allowing enzymes to act on substrates at a considerably-high concentration because the enzymatic reaction per se is an equilibrium reaction of 2 different types of enzymes and the equilibrium point constantly inclines to the side of forming glucose phosphate.

In view of the foregoing, the present inventors energetically screened enzymes which directly convert maltose into trehalose, and have found that microorganisms including *Pimelobacter* sp. R48, as disclosed in EP-A-0 636 693 and in JP-A-170,977/95, produce an absolutely novel enzyme which forms trehalose when acts on maltose. This means that trehalose can be prepared from maltose as a material which is readily available in quantity and at low cost, and all the above mentioned objects would be completely overcome thereby. The enzyme productivity of these microorganisms, however, is not sufficient, and this necessitates a considerably-large scale cultivation to obtain an industrial-scale production of trehalose.

Recombinant DNA technology has made a remarkable progress in recent years. At present, even an enzyme, whose total amino acid sequence has not been revealed, can be readily prepared in a desired amount, if a gene encoding the enzyme was once isolated and the base sequence was decoded, by preparing a recombinant DNA containing a DNA which encodes the enzyme, introducing the recombinant DNA into microorganisms or cells of plants or animals, and culturing the resultant transformants. Under these circumstances, urgently required are the finding of a gene which encodes an enzyme capable of forming trehalose from maltose, and the elucidation of the base sequence.

It is an aim of the present invention to provide a DNA encoding a recombinant enzyme which forms trehalose when acts on maltose.

It is a further aim of the present invention to provide a replicable recombinant DNA containing the DNA.

It is a further aim of the present invention to provide a transformant into which the recombinant DNA has been introduced.

It is a further aim of the present invention to provide a process for preparing the recombinant enzyme by using the transformant.

It is a further aim of the present invention to provide a method for converting maltose into trehalose by the recombinant enzyme prepared by using the transformant.

The present invention provides an isolated DNA which encodes a recombinant enzyme having the following physiochemical properties:
(1) Action
   Forming trehalose when acts on maltose, and *vice versa;*
(2) Molecular weight
   57,000-67,000 daltons when assayed on sodium dodecyl sulfate polyacrylamide gel electrophoresis (SDS-PAGE); and
(3) Isoelectric point (pI)
   4.1-5.1 when assayed on isoelectrophoresis;
and wherein the base sequence of the DNA comprises (i) the base sequence of SEQ ID NO:2, (ii) a complementary base sequence thereto, or (iii) a variant of the base sequence of SEQ ID NO:2 where one or more bases in SEQ ID NO:2 are replaced with other bases by means of the degeneracy of genetic code, or one or more bases of SEQ ID NO: 2 are replaced with other bases without altering the inherent activity of the recombinant enzyme.

The present invention further provides a replicable recombinant DNA which contains the DNA and a self-replicable vector.

The present invention further provides a transformant obtained by introducing the replicable recombinant DNA into an appropriate host.

The present invention further provides a process comprising culturing the transformant in a nutrient culture medium to form the recombinant enzyme, and collecting the formed recombinant enzyme from the resultant culture.

The present invention further provides an enzymatic conversion method of maltose which contains a step of allowing the recombinant enzyme, prepared by using the transformant, to act on maltose to form trehalose.

The invention will now be described in further detail, by way of example only, with reference to the accompanying drawings in which:-
FIG. 1 shows the optimum temperature of an enzyme produced by *Pimelobacter* sp. R48.
FIG. 2 shows the optimum pH of an enzyme produced by *Pimelobacter* sp. R48.
FIG. 3 shows the thermal stability of an enzyme produced by *Pimelobacter* sp. R48.
FIG. 4 shows the pH stability of an enzyme produced by *Pimelobacter* sp. R48.
FIG. 5 shows the structure of the recombinant DNA pBRM8 according to the present invention.

The recombinant enzyme according to the present invention forms trehalose when acts on maltose.

The DNA according to the present invention expresses the formation of the present recombinant enzyme by introducing the DNA into an appropriate self-replicable vector to obtain a replicable recombinant DNA which is then introduced into an appropriate host, inherently incapable of forming the recombinant enzyme but readily proliferative, to form a transformant.

The recombinant DNA according to the present invention expresses the production of the recombinant enzyme by introducing the DNA into an appropriate host, which is inherently incapable of forming the recombinant enzyme but readily proliferative, to form a transformant, and culturing the transformant in a nutrient culture medium.

The transformant according to the present invention forms the recombinant enzyme when cultured.

The recombinant enzyme according to the present invention can be formed in a desired amount and with a relative easiness by the process disclosed in the present specification.

The enzymatic conversion method according to the present invention converts maltose into trehalose with a relative easiness.

Experiments for revealing the physicochemical properties of an enzyme produced by *Pimelobacter* sp. R48 are as follows:

### Experiment 1

### Purification of enzyme

### Experiment 1-1

### Production of enzyme

In 500-ml Erlenmeyer flasks were placed 100 ml aliquots of a liquid culture medium (pH 7.2) containing 2.0 w/v % glucose, 0.5 w/v % polypeptone, 0.1 w/v % yeast extract, 0.1 w/v % disodium hydrogen phosphate, 0.06 w/v % potassium dihydrogen phosphate, 0.05 w/v % magnesium sulfate heptahydrate, 0.5 w/v % calcium carbonate, and water, and the flasks were autoclaved at 115°C for 30 min to effect sterilization. After cooling the flasks a seed culture of *Pimelobacter* sp. R48 was inoculated into each flask, followed by the incubation at 27°C for 24 hours under a rotary-shaking condition of 200 rpm. Twenty L aliquots of a fresh preparation of the same liquid culture medium were put in 30-L jar fermenters and sterilized, followed by inoculating one v/v % of the culture obtained in the above into each liquid culture medium, and incubating the resultant at a pH of 6.0-8.0 and 27°C for about 40 hours under aeration-agitation conditions.

Thereafter, the enzymatic activity of the resultant culture was assayed to reveal that it contained about 0.55 units/ml of the enzyme. A portion of the culture was centrifuged, and the supernatant was assayed to reveal that it contained about 0.05 units/ml of the enzyme. While the separated cells were suspended in 50 mM phosphate buffer (pH 7.0) to give the initial volume of the portion, followed by assaying the suspension to reveal that it contained about 0.5 units/ml of the enzyme.

Throughout the specification the enzyme activity is expressed by the value measured on the following assay: Place one ml of 10 mM phosphate buffer (pH 7.0) containing 20 w/v % maltose in a test tube, add one ml of an appropriately diluted enzyme solution to the tube, and incubate the solution in the tube at 25°C for 60 min to effect an enzymatic reaction, followed by further incubation at 100°C for 10 min to suspend the enzymatic reaction. Thereafter, a portion of the reaction mixture was diluted by 11 times with 50 mM phosphate buffer (pH 7.5), and 0.4 ml of which was placed in a test tube, admixed with 0.1 ml solution containing one unit/ml trehalase, followed by incubating the resultant mixture at 45°C for 120 min and quantifying the glucose content on the glucose oxidase method. As a control, a system, comprising a trehalase solution and an enzyme solution which has been inactivated by heating at 100°C for 10 min, is provided and treated similarly as above. The content of the formed trehalose can be estimated based on the glucose content quantified in the above. One unit of the enzymatic activity is defined as the amount which forms one µmol trehalose per min under the above conditions.

### Experiment 1-2

### Purification of enzyme

The culture obtained in Experiment 1-1 was centrifuged to separate cells, and about 0.5 kg of the wet cells thus obtained was suspended in 10 mM phosphate buffer (pH 7.0), disrupted in usual manner, and centrifuged to obtain an about 4.5 L of a crude enzyme solution. To the solution was added ammonium sulfate to give a saturation of 30 w/v %, salted out by standing it at 4°C for 4 hours, and centrifuged to obtain a supernatant. Ammonium sulfate was added to the supernatant to give a saturation of 80 w/v %, and allowed to stand it at 4°C overnight. The resultant sediment was collected by centrifugation, dissolved in a small amount of 10 mM phosphate buffer (pH 7.0), and dialyzed against 10 mM phosphate buffer (pH 7.0) for 24 hours. The dialyzed inner solution was centrifuged to obtain a supernatant which was then applied to a column packed with "DEAE-TOYOPEARL®", a column for ion-exchange chromatography commercialized by Tosoh Corporation, Tokyo, Japan, which had been previously equilibrated with 10 mM phosphate buffer (pH 7.0), followed by feeding to the column a linear gradient buffer of sodium chloride ranging from 0 M to 0.4 M in 10 mM phosphate buffer (pH 7.0). From the eluate fractions with the objective enzyme activity were collected, pooled, dialyzed against 10 mM phosphate buffer (pH 7.0) containing 1 M ammonium sulfate for 10 hours, and centrifuged to obtain a supernatant. The supernatant thus obtained was applied to a column packed with "BUTYL-TOYOPEARL®", a column for hydrophobic chromatography commercialized by Tosoh Corporation, Tokyo, Japan, which had been previously equilibrated with 10 mM phosphate buffer (pH 7.0) containing 1 M ammonium sulfate, followed by feeding to the column a linear gradient buffer of ammonium sulfate ranging from 1 M to 0 M in 10 mM phosphate buffer (pH 7.0). From the eluate fractions with the objective enzyme activity were collected, pooled and applied to a column packed with "MONO Q HR5/5", a column for ion-exchange chromatography commercialized by Pharmacia LKB Biotechnology AB Uppsala, Sweden, which had been previously equilibrated with 10 mM phosphate buffer (pH 7.0), followed by feeding to the column a linear gradient buffer of sodium chloride ranging from 0 M to 0.5 M, and collecting fractions with the enzyme activity from the eluate. The resultant purified enzyme had a specific activity of about 17 units/mg protein, and the yield was about 46 units per L of the culture.

The purified enzyme was electrophoresed on 7.5 w/v % polyacrylamide gel to give a single protein band with an active enzyme, and this meant that it was considerably-high in purity.

### Experiment 2

### Physicochemical property of enzyme

### Experiment 2-1

### Action

To an aqueous solution containing 5 w/v % maltose or trehalose as a substrate was added 2 units/g substrate of the purified enzyme obtained in Experiment 1-2, and the mixture was incubated at 20°C and pH 7.0 for 24 hours. In order to analyze the saccharide composition of the reaction mixture, it was dried *in vacuo*, dissolved in pyridine, and trimethylsylated in usual manner, and the resultant was subjected to gas chromatography. The equipments and conditions used in this analysis were as follows: "GC-16A" commercialized by Shimadzu Seisakusho, Ltd., Tokyo, Japan, as a gas chromatograph; a stainless steel column, having an inner diameter of 3 mm and a length of 2 m, packed with 2% "SILICONE OV-17/CHROMOSOLB W" commercialized by GL Sciences Inc., Tokyo, Japan, as a column; a model of hydrogen flame ionization as a detector; nitrogen gas as a carrier gas (flow rate of 40 ml/min); and a column temperature of 160-320°C at a programmed temperature rate of 7.5°C/min. The saccharide compositions of the reaction mixtures were tabulated in Table 1:

**Table 1**

| Substrate | Saccharide in reaction mixture | Saccharide composition (w/w %) |
|---|---|---|
| Maltose | Glucose | 4.9 |
| | Maltose | 22.3 |
| | Trehalose | 72.8 |
| Trehalose | Glucose | 3.2 |
| | Maltose | 17.2 |
| | Trehalose | 79.6 |

As shown in Table 1, the purified enzyme formed about 73 w/w % trehalose and about 5 w/w % maltose when acted on maltose as a substrate, while it formed about 17 w/w % maltose and about 3 w/w % glucose when acted on trehalose as a substrate. These facts indicate that the purified enzyme has activities of converting maltose into trehalose and of converting trehalose into maltose, as well as of hydrolyzing α-1,4 linkage in maltose molecule and α,α-1,1 linkage in trehalose molecule. There has been no report of such an enzyme, and this led to an estimation of having a novel enzymatic pathway.

### Experiment 2-2

### Molecular weight

In accordance with the method reported by U. K. Laemmli in *Nature*, Vol.227, pp.680-685 (1970), the purified enzyme was electrophoresed on sodium dodecyl sulfate polyacrylamide gel electrophoresis (SDS-PAGE) to show a single protein band at a position corresponding to about 57,000-67,000 daltons. The marker proteins used in this experiment were myosin (MW=200,000 daltons), β-galactosidase (MW=116,250 daltons), phosphorylase B (MW=97,400 daltons), serum albumin (MW=66,200 daltons) and ovalbumin (MW=45,000 daltons).

### Experiment 2-3

### Isoelectric point

The purified enzyme gave an isoelectric point of about 4.1-5.1 when measured on isoelectrophoresis.

### Experiment 2-4

### Optimum temperature

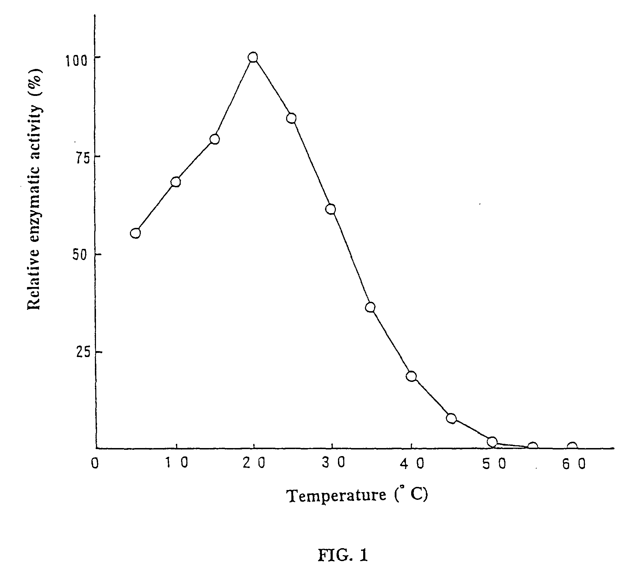

The optimum temperature of the purified enzyme was about 20°C as shown in FIG. 1 when incubated in usual manner in 10 mM phosphate buffer (pH 7.0) for 60 min.

### Experiment 2-5

### Optimum pH

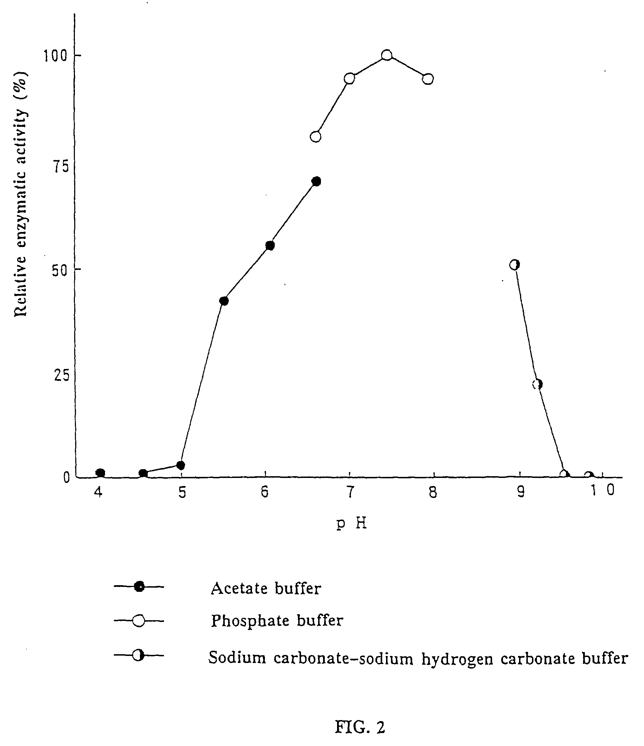

The optimum pH of the purified enzyme was about 7.0-8.0 as shown in FIG. 2 when experimented in usual manner by incubating it at 25°C for 60 min in 10 mM acetate buffer, phosphate buffer or sodium carbonate-sodium hydrogen carbonate buffer having different pHs.

### Experiment 2-6

### Thermal stability

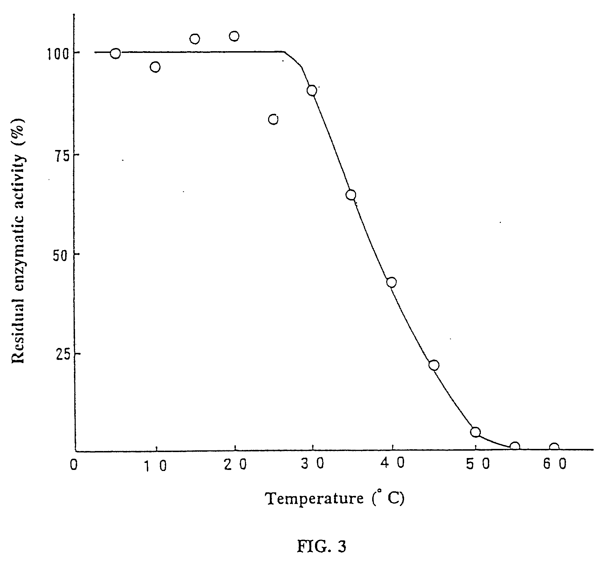

The purified enzyme was stable up to a temperature of about 30°C as shown in FIG. 3 when experimented in usual manner by incubating it in 50 mM phosphate buffer (pH 7.0) for 60 min.

### Experiment 2-7

### pH Stability

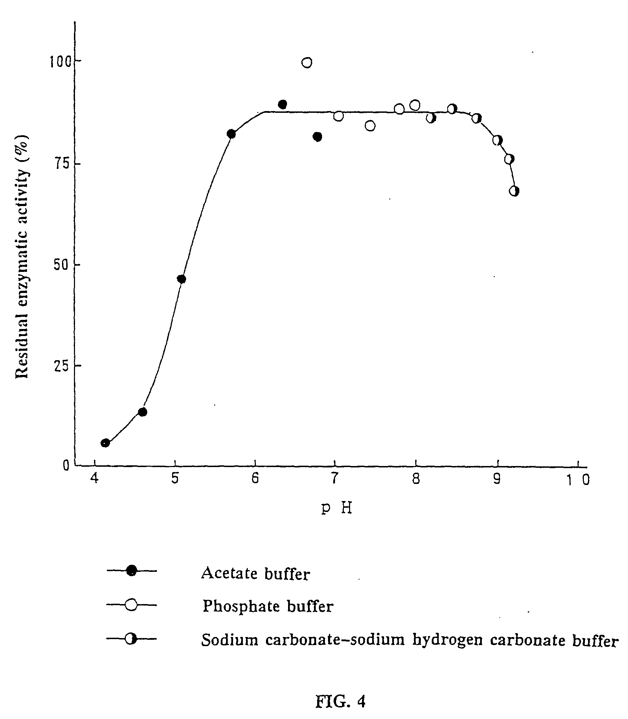

The purified enzyme was stable up to a pH of about 6.0-9.0 as shown in FIG. 4 when experimented in usual manner by incubating it at 20°C for 60 min in 50 mM acetate buffer, phosphate buffer or sodium carbonate-sodium hydrogen carbonate buffer having different pHs.

### Experiment 2-8

### Amino acid sequence containing the N-terminal

The amino acid sequence containing the N-terminal of the purified enzyme was analyzed on "MODEL 470A", a gas-phase protein sequencer commercialized by Perkin-Elmer Corp., Norwalk, USA, to reveal that it has an amino acid sequence containing the N-terminal as shown in SEQ ID NO:3.

### Experiment 2-9

### Partial amino acid sequence

An adequate amount of the purified enzyme prepared in Experiment 1 was weighed, dialyzed against 10 mM Tris-HCl buffer (pH 9.0) at 4°C for 18 hours, and admixed with 10 mM Tris-HCl buffer (pH 9.0) to obtain a solution containing about one mg/ml of the enzyme. About one ml of the solution was placed in a test tube, admixed with 10 µg lysyl endopeptidase, and incubated at 30°C for 22 hours to partially hydrolyze the enzyme. The resultant hydrolysate was applied to "µBONDAPAK C18", a column for reverse-phase high-performance liquid chromatography commercialized by Japan Millipore Ltd., Tokyo, Japan, which had been previously equilibrated with 0.1 v/v % trifluoroacetate containing 16 v/v % aqueous acetonitrile, followed by feeding to the column 0.1 v/v % trifluoroacetate containing acetonitrile at a flow rate of 0.9 ml/min while increasing the concentration of acetonitrile from 16 v/v % to 44 v/v % and separately collecting fractions containing a peptide fragment, eluted about 46 min after the initiation of the feeding. Fractions containing the peptide fragment were pooled, dried in vacuo, and dissolved in 0.1 v/v % trifluoroacetate containing 50 v/v % aqueous acetonitrile. Similarly as in Experiment 2-8, the peptide fragment was analyzed and revealed to have an amino acid sequence as shown in SEQ ID NO:4.

No enzyme having these physicochemical properties has been known, and this concluded that it is a novel substance. Referring to *Pimelobacter* sp. R48, it is a microorganism which was isolated from a soil in Okayama-city, Okayama, Japan, deposited on June 3, 1993, in National Institute of Bioscience and Human-Technology Agency of Industrial Science and Technology, Tsukuba, Ibaraki, Japan, and accepted under the accession number of FERM BP-4315, and it has been maintained by the institute. The bacteriological characteristics of the microorganism are disclosed in detail in JP-A-170,977/95 applied by the present applicant.

The present inventors energetically screened the chromosomal DNA of *Pimelobacter* sp. R48 by using an oligonucleotide as a probe which had been chemically synthesized based on the amino acid sequence containing the N-terminal and the partial amino acid sequence as revealed in Experiments 2-8 and 2-9, and have obtained a DNA fragment which consists of about 1,700 base pairs having the base sequence as shown in the following SEQ ID NO:2 that initiates from the 5'-terminus. The decoding of the base sequence revealed that it consists of 568 amino acids and has the amino acid sequence that initiates from the N-terminal as shown in SEQ ID NO:1.

The sequential experimental steps, used to reveal the base sequence, and amino acid sequence as shown in SEQ ID NOs:1 and 2 are summarized in the below:
(1) The enzyme was isolated from a culture of a donor microorganism, highly purified, and determined for its amino acid sequence containing the N-terminal. The purified enzyme was partially hydrolyzed with protease, and from which a peptide fragment was isolated and determined for its amino acid sequence;
(2) Separately, a chromosomal DNA was isolated from a donor microorganism's cell, purified and partially digested by a restriction enzyme to obtain a DNA fragment consisting of about 2,000-6,000 base pairs. The DNA fragment was ligated by a DNA ligase to a plasmid vector, which had been previously cut with a restriction enzyme, to obtain a recombinant DNA;
(3) The recombinant DNA was introduced into a microorganism of the species *Escherichia coli* to obtain transformants, and from which an objective transformant containing a DNA encoding the enzyme was selected by the colony hybridization method using an oligonucleotide, as a probe, which had been chemically synthesized based on the aforesaid partial amino acid sequence; and
(4) The recombinant DNA was obtained from the selected transformant and annealed with a primer, followed by allowing a DNA polymerase to act on the resultant to extend the primer, and determining the base sequence of the resultant complementary chain DNA by the dideoxy chain termination method. The comparison of an amino acid sequence, which is estimable from the determined base sequence, with the aforesaid amino acid sequence concluded that it encodes the enzyme.

The following Experiments 3 and 4 concretely illustrate the above items (2) to (4), and the techniques used in these experiments were conventional ones commonly used in this field, for example, those described by J. Sumbruck et al. in "*Molecular Cloning A Laboratory Manual*", 2nd edition, published by Cold Spring Harbor Laboratory Press (1989).

### Experiment 3

### Preparation of recombinant DNA containing DNA derived from Pimelobacter sp. R48 and preparation of transformant

### Experiment 3-1

### Preparation of chromosomal DNA

A seed culture of *Pimelobacter* sp. R48 was inoculated into bacto nutrient broth medium (pH 7.0), and cultured at 27°C for 24 hours with a rotary shaker. The cells were separated from the resultant culture by centrifugation, suspended in TES buffer (pH 8.0), admixed with 0.05 w/v % lysozyme, and incubated at 37°C for 30 min. The resultant was freezed at -80°C for one hour, admixed with TSS buffer (pH 9.0), heated to 60°C, and further admixed with a mixture solution of TES buffer and phenol, and the resultant solution was chilled with ice, followed by centrifugation to obtain a supernatant. To the supernatant was added 2-fold volumes of cold ethanol, and the precipitated crude chromosomal DNA was collected, suspended in SSC buffer (pH 7.1), admixed with 7.5 µg ribonuclease and 125 µg protease, and incubated at 37°C for one hour. Thereafter, a mixture solution of chloroform and isoamyl alcohol was added to the reaction mixture to extract the objective chromosomal DNA, and the extract was admixed with cold ethanol, followed by collecting the formed sediment containing the chromosomal DNA. The resultant purified chromosomal DNA was dissolved in SSC buffer (pH 7.1) to give a concentration of about one mg/ml, and the resultant solution was freezed at -80°C.

### Experiment 3-2

### Preparation of recombinant DNA pBRM8 and transformant BRM8

About one ml of the purified chromosomal DNA obtained in Example 3-1 was placed in a test tube, admixed with about 35 units of *Sau* 3AI, a restriction enzyme, and enzymatically reacted at 37°C for about 20 min to partially digest the chromosomal DNA, followed by recovering a DNA fragment consisting of about 2,000-6,000 base pairs by means of sucrose density-gradient ultracentrifugation. One µg of Bluescript II SK(+), a plasmid vector, was placed in a test tube, subjected to the action of *Bam* HI, a restriction enzyme, to completely digest the plasmid vector, admixed with 10 µg of the DNA fragment and 2 units of T4 DNA ligase, and allowed to stand at 4°C overnight to ligate the DNA fragment to the plasmid vector fragment. To the resultant recombinant DNA was added 30 µl of "Epicurian Coli® XLI-Blue", a competent cell commercialized by Toyobo Co., Ltd., Tokyo, Japan, allowed to stand under ice-chilling conditions for 30 min, heated to 42°C, admixed with SOC broth, and incubated at 37°C for one hour to introduce the recombinant DNA into *Escherichia coli*.

The resultant transformant was inoculated into agar plate (pH 7.0) containing 50 pg/ml of 5-bromo-4-chloro-3-indolyl-β-galactoside, and cultured at 37°C for 18 hours, followed by placing a nylon film on the agar plate to fix thereon about 6,000 colonies formed on the agar plate. Based on the amino acid sequence located at positions from 6 to 11, i.e. Glu-Glu-Pro-Glu-Trp-Phe, the base sequence of probe 1 as shown in SEQ ID NO:6 was chemically synthesized, labelled with ³²P, and hybridized with the colonies of transformants fixed on the nylon film, followed by selecting 5 transformants which had strongly hybridized with the probe 1.

The objective recombinant DNA was selected in usual manner from the 5 transformants, and, in accordance with the method described by E. M. Southern in *Journal of Molecular Biology*, Vol.98, pp.503-517 (1975), the recombinant DNA was hybridized with probe 2 having the base sequence as shown in SEQ ID NO:7, which had been chemically synthesized based on the amino acid sequence located at positions from 5 to 10, i.e. Met-Leu-Glu-Ala-Met-Ala, followed by selecting a recombinant DNA which had strongly hybridized with the probe 2. The recombinant DNA and transformant thus selected were respectively named "pBRM8" and "BRM8".

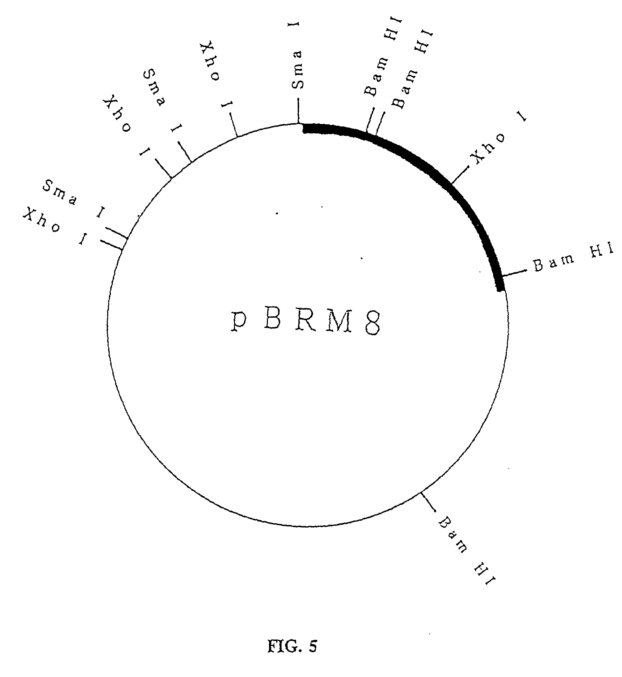

The transformant BRM8 was inoculated into L-broth (pH 7.0) containing 100 µg/ml ampicillin, and cultured at 37°C for 24 hours by a rotary shaker. After completion of the culture, the resultant cells were centrifugally collected from the culture, and treated with the alkaline method in general to extracellularly extract a recombinant DNA. The extract was in usual manner purified and analyzed to reveal that the recombinant DNA pBRM8 consists of about 7,600 base pairs and that as shown in FIG. 5 the DNA, which consists of about 1,700 base pairs and encodes the enzyme, is positioned in the downstream near to the digested site of *Sma* I, a restriction enzyme.

### Experiment 3-3

### Production of recombinant enzyme by transformant BRM8

In 500-ml flasks were placed 100 ml aliquots of a liquid nutrient culture medium consisting of 2.0 w/v % glucose, 0.5 w/v % peptone, 0.1 w/v % yeast extract, 0.1 w/v % dipotassium hydrogen phosphate, 0.06 w/v % sodium dihydrogen phosphate, 0.05 w/v % magnesium sulfate heptahydrate, 0.5 w/v % calcium carbonate and water, and each flasks was sterilized by heating at 115°C for 30 min, cooled, admixed with 50 µg/ml ampicillin, and inoculated with the transformant BRM8 obtained in Experiment 3-2, followed by culturing the transformant at 37°C for 24 hours by a rotary shaker. The resultant culture was treated with an ultrasonic disintegrator to disrupt cells, and the resultant suspension was centrifuged to remove insoluble substances. The supernatant thus obtained was assayed for the enzyme activity to reveal that one L of the culture contained about 850 units of the enzyme.

As a control, a seed culture of *Escherichia coli* XLI-Blue or *Pimelobacter* sp. R48 was inoculated in a fresh preparation of the same liquid nutrient culture medium but free of ampicillin, and, in the case of culturing *Pimelobacter* sp. R48, it was cultured and treated similarly as above except that the cultivation temperature was set to 27°C. Assaying the resultant activity, one L culture of *Pimelobacter* sp. R48 contained about 410 units of the enzyme, and the yield was significantly lower than that of transformant BRM8. *Escherichia coli* XLI-Blue used as a host did not form the enzyme.

Thereafter, the enzyme produced by the transformant BRM8 was purified similarly as in Experiment 1-2, and examined on its physicochemical properties and characteristics. As a result, it was revealed that it has substantially the same physicochemical properties as the enzyme of *Pimelobacter* sp. R48, i.e. it has a molecular weight of about 57,000-67,000 daltons on SDS-PAGE and an isoelectric point of about 4.1-5.1 on isoelectrophoresis. The results indicate that the present enzyme can be prepared by the recombinant DNA technology, and the yield can be significantly increased thereby.

### Experiment 4

### Preparation of complementary chain DNA and determination for its base sequence and amino acid sequence

Two µg of the recombinant DNA pBRM8 obtained in Experiment 3-2 was placed in a test tube, admixed with 2 M aqueous sodium hydroxide solution to effect degeneration, and admixed with an adequate amount of cold ethanol, followed by collecting the formed sediment containing a template DNA and drying the sediment *in vacuo*. To the template DNA were added 50 pmole/ml of a chemically synthesized primer 1 represented by the following SEQ ID NO:8, 10 µl of 40 mM Tris-HCl buffer (pH 7.5) containing 20 mM magnesium chloride and 20 mM sodium chloride, and the mixture was incubated at 65°C for 2 min to effect annealing and admixed with 2 µl of an aqueous solution containing dATP, dGTP and dTTP in respective amounts of 7.5 µM, 0.5 µl of [α-³²P]dCTP (2 mCi/ml), one µl of 0.1 M dithiothreitol, and 2 µl of 1.5 units/ml T7 DNA polymerase, followed by incubating the resultant mixture at 25°C for 5 min to extend the primer 1 from the 5'-terminus to the 3'-terminus. Thus, a complementary chain DNA was formed.

The reaction product containing the complementary chain DNA was divided into four equal parts, to each of which 2.5 µl of 50 mM aqueous sodium chloride solution containing 80 µM dNTP and 8 µM ddATP, ddCTP, ddGTP or ddTTP was added, and the resultant mixture was incubated at 37°C for 5 min, followed by suspending the reaction by the addition of 4 µl of 98 v/v % aqueous formamide solution containing 20 mM EDTA, 0.05 w/v % bromophenol blue, and 0.05 w/v % xylene cyanol. The reaction mixture was heated with a boiling-water bath for 3 min, and a small portion of which was placed on a 6 w/v % polyacrylamide gel, and electrophoresed by energizing it with a constant voltage of about 2,000 volts to separate DNA fragments, followed by fixing the gel in usual manner, drying it and subjecting the resultant to autoradiography.

Analyses of the DNA fragments separated on the radiogram revealed that the complementary chain DNA contains the base sequence consisting of about 1,700 base pairs as shown in SEQ ID NO:5. An amino acid sequence estimable from the base sequence was shown in parallel in SEQ ID NO:5 and compared with the amino acid sequence containing the N-terminal or the partial amino acid sequence, which had been revealed in Experiments 2-8 and 2-9, to reveal that the amino acid sequence containing the N-terminal as shown in Experiment 2-8 corresponded to the amino acid sequence located at positions from 1 to 20 in SEQ ID NO:5, and that the partial amino acid sequence as shown in Experiment 2-9 corresponded to the amino acid sequence located at positions from 183 to 193. These results indicate that the present enzyme has the amino acid sequence as shown in SEQ ID NO:1, and the enzyme of *Pimelobacter* sp. R48 is encoded by the DNA having the base sequence as shown in SEQ ID NO:2.

As is described above, the present inventors found the enzyme which converts maltose into trehalose and vice versa as a result of their long-term research, and it has a specific physicochemical properties differing from conventional enzymes. The present invention aims to prepare a recombinant enzyme by means of recombinant DNA technology. With reference to examples, the process for preparing such a recombinant enzyme and its uses are described in detail.

The recombinant enzyme as referred to in the present invention includes those which are prepared by the recombinant DNA technology and capable of converting maltose into trehalose and *vice versa*. Usually the present recombinant DNA has a revealed amino acid sequence, e.g. the amino acid sequence as shown in SEQ ID NO:1 or a complementary amino acid to the amino acid sequence. Variants containing amino acid sequences which are homologous to the amino acid sequence of SEQ ID NO:1 can be prepared by replacing one or more amino acids in those in SEQ ID NO:1 with other amino acids without altering the inherent activity of the enzyme. Although even when used the same DNA and it also depends on hosts into which the DNA is introduced, as well as on ingredients and components of nutrient culture media used for culturing transformants, and their cultivation temperature and pH, there may be produced modified enzymes which have the enzymatic activity inherent to the enzyme encoded by the DNA but defect one or more amino acids located in nearness to the N-terminal of the amino acid sequence of SEQ ID NO:1, or have one or more amino acids newly added to the N-terminal by the modification of intracellular enzymes of hosts after the DNA expression. Such variants can be included in the present recombinant enzyme as long as they convert maltose into trehalose and *vice versa*.

The recombinant enzyme according to the present invention can be obtained from cultures of transformants containing the specific DNA. Transformants usable in the present invention can be obtained by introducing into appropriate hosts the base sequence containing the 5'-terminus as shown in SEQ ID NO:2, homologous base sequences, or complementary base sequences to these base sequences. One or more bases in the above mentioned base sequences may be replaced with other bases by means of degeneracy of genetic code without altering the amino acid sequence which they encode. Needless to say, one or more bases in the base sequence, which encodes the enzyme or their variants, can be readily replaced with other bases to allow the DNA to actually express the enzyme production in hosts.

Any DNA derived from natural resources and those artificially synthesized can be used in the present invention as long as they have the aforementioned base sequences. The natural resources of the DNA according to the present invention are, for example, microorganisms of the genus *Pimelobacter*, i.e. *Pimelobacter* sp. R48, from which genes containing the present DNA can be obtained. These microorganisms can be inoculated in nutrient culture media and cultured for about 1-3 days under aerobic conditions, and the resultant cells were collected from cultures and subjected to ultrasonication or treated with a cell-wall lysis enzyme such as lysozyme or β-glucanase to extract genes containing the present DNA. In this case, a proteolytic enzyme such as protease can be used in combination with the cell-wall lysis enzyme, and, in the case of treating the cells with ultrasonication, they may be treated in the presence of a surfactant such as sodium dodecyl sulfate (SDS) or treated with freezing and thawing method. The objective DNA is obtainable by treating the resultant with phenol extraction, alcohol sedimentation, centrifugation, protease treatment and/or ribonuclease treatment used in general in this field. T o artificially synthesize the DNA according to the present invention, it can be chemically synthesized by using the base sequence as shown in SEQ ID NO:2, or can be obtained in plasmid form by inserting a DNA, which encodes the amino acid sequence as shown in SEQ ID NO:1, into an appropriate self-replicable vector to obtain a recombinant DNA, introducing the recombinant DNA into an appropriate host to obtain a transformant, culturing the transformant, separating the proliferated cells from the resultant culture, and collecting plasmids containing the recombinant DNA from the cells.

Such a recombinant DNA is usually introduced in the form of a recombinant DNA into hosts. Generally the recombinant DNA contains the aforesaid DNA and a self-replicable vector and it can be prepared by conventional method with a relative easiness when the material DNA is in hand. Examples of such a vector are plasmid vectors such as pBR322, pUC18, Bluescript II SK(+), pUB110, pTZ4, pC194, pHV14, TRp7, TEp7, pBS7, etc.; and phage vectors such as λgt·λC, λgt·λB, ρ11, φ1, φ105, etc. Among these plasmid- and phage-vectors, pBR322, pUC18, Bluescript II SK(+), λgt·λC and λgt·λB are satisfactorily used in case that the present DNA should be expressed in *Escherichia coli*, while pUB110, pTZ4, pC194, pll, φ1 and φ105 are satisfactorily used to express the DNA in microorganisms of the genus *Bacillus*. The plasmid vectors pHV14, TRp7, TEp7 and pBS7 are suitably used when the recombinant DNA is allowed to grow in 2 or more hosts.

The methods used to insert the present DNA into such vectors in the present invention may be conventional ones generally used in this field. A gene containing the present DNA and a self-replicable vector are first digested by a restriction enzyme and/or ultrasonic disintegrator, then the resultant DNA fragments and vector fragments are ligated. To ligate DNA fragments and vectors, firstly they may be annealed if necessary, then subjected to the action of a DNA ligase *in vivo* or *in vitro*. The recombinant DNA thus obtained is replicable without substantial limitation by introducing it into an appropriate host, and culturing the resultant transformant.

The recombinant DNA according to the present invention can be introduced into appropriate host microorganisms including *Escherichia coli* and those of the genus *Bacillus* as well as actinomyces and yeasts. In the case of using *Escherichia coli* as a host, it can be cultured in the presence of the recombinant DNA and calcium ion, while in the case of using the microorganisms of the genus *Bacillus* the competent cell method and the colony hybridization method can be employed. Desired transformants can be cloned by the colony hybridization method or by culturing a variety of transformants in nutrient culture media containing either maltose or trehalose and selecting transformants which form trehalose or maltose.

The transformants thus obtained extracellularly produce the objective enzyme when cultured in nutrient culture media. Generally, liquid media in general supplemented with carbon sources, nitrogen sources and/or minerals, and, if necessary, further supplemented with a small amount of amino acids and/or vitamins can be used as the nutrient culture media. Examples of the carbon sources are saccharides such as starch, starch hydrolysate, glucose, fructose and sucrose. Examples of the nitrogen sources are organic- and inorganic-substances containing nitrogen such as ammonia, ammonium salts, urea, nitrate, peptone, yeast extract, defatted soy been, corn steep liquor and beef extract. Cultures containing the objective enzyme can be obtained by inoculating the transformants into nutrient culture media, and incubating them at a temperature of 20-50°C and a pH of 2-9 for about 1-6 days under aerobic aeration-agitation conditions. Such cultures can be used intact as a crude enzyme preparation, and, usually, cells in the cultures can be disrupted with ultrasonic disintegrator and/or cell-wall lysis enzymes prior to use, followed by separating the enzyme from intact cells and cell debris by filtration and/or centrifugation, and purifying the enzyme. The methods used for purifying the enzyme in the invention include conventional ones in general. From cultures intact cells and cell debris are removed and subjected to one or more methods such as concentration, salting out, dialysis, separately sedimentation, gel filtration chromatography, ion-exchange chromatography, hydrophobic chromatography, affinity chromatography, gel electrophoresis and isoelectric point electrophoresis.

As is described above, the present recombinant enzyme exerts a distinct activity of forming trehalose or maltose from maltose or trehalose respectively, and such an activity has not been found in conventional enzymes. Trehalose has a mild and high-quality sweetness and it has a great advantage of being capable of sweetening food products without fear of causing unsatisfactorily coloration and deterioration because it has no reducing residue within the molecule. By using these properties of the present recombinant enzyme, maltose, which could not have been used in some field due to its reducibility, can be converted into useful trehalose with a satisfactory handleability and substantial no reducibility.

To effectively produce trehalose in an industrial scale, saccharide compositions with a relatively-high maltose content, i.e., usually, about 70 w/w % or more, preferably, about 80 w/w % or more, can be arbitrarily used. Such saccharide compositions can be prepared by conventional methods generally used in this field, for example, those as disclosed in JP-B-11,437/81 and JP-B-17,078/81 wherein β-amylase is allowed to act on gelatinized-or liquefied-starch and separating the formed maltose by separation-sedimentation method or dialysis method, or those as disclosed in JP-B-13,089/72 and JP-B-3,938/79
wherein β-amylase is allowed to act on gelatinized- or liquefied-starch together with a starch debranching enzyme such as isoamylase or pullulanase.

In the enzymatic conversion method according to the present invention, an effective amount of the present recombinant enzyme is allowed to coexist in an aqueous medium containing maltose, followed by keeping the resultant mixture at a prescribed temperature and pH to enzymatically react until the desired amount of trehalose is formed. Although the enzymatic reaction proceeds even at a relatively-low concentration of about 0.1 w/w %, d.s.b., the concentration may be set to about 2 w/w % or more, d.s.b., preferably, about 5-50 w/w %, d.s.b., to proceed the enzymatic conversion method in an industrial scale. The reaction temperature and pH are set within the range which effectively forms maltose without inactivating the recombinant enzyme, i.e. a temperature of about 30°C, preferably, about 4-30°C, and a pH of about 6-9, preferably, about 7-8. The amount of the recombinant enzyme and the reaction time are appropriately set depending on the conditions of the enzymatic reaction. The present enzymatic conversion method effectively converts maltose into trehalose, and the conversion rate reaches up to about 80% or more in some cases.

The reaction mixtures obtainable by the present enzymatic conversion method can be used intact, and, usually, they may be purified prior to use. For example, the reaction mixtures are filtered and centrifuged to remove insoluble substances, and the resultant solutions are decolored with an activated charcoal, desalted and purified with an ion-exchange resin, and concentrated into syrupy products. Depending on use, the syrupy products can be dried *in vacuo* and spray-dried into solid products. To obtain products substantially consisting of trehalose, the syrupy products are subjected to one or more methods of chromatographies using ion exchangers, activated charcoals or silica gels, fermentation using yeasts, and removal by decomposing reducing saccharides with alkalis. To treat a relatively-large amount of reaction mixtures, ion-exchange chromatographies such as fixed bed-, moving bed-, and pseudo-moving bed-methods as disclosed in JP-A-23,799/83 and JP-A-72,598/88 are arbitrarily used in the invention, and these enable an industrial-scale production of high-trehalose content products, which have been difficult to obtain in large quantities, in a considerably-high yield. The trehalose and saccharide compositions containing trehalose thus obtained can be used in a variety of products which should be avoided from the reducibility of saccharide sweeteners, and therefore, they can be arbitrarily used in food products in general, cosmetics and pharmaceuticals as a sweetener, taste-improving agent, quality-improving agent, stability, filler, adjuvant or excipient.

The following examples explain the preparation of the recombinant enzyme and the enzymatic conversion method of maltose according to the present invention:

### Example A-1

### Preparation of recombinant enzyme

To 500-ml Erlenmeyer flasks were added 100 aliquots of a nutrient culture medium consisting of 2.0 w/v % glucose, 0.5 w/v % peptone, 0.1 w/v % yeast extract, 0.1 w/v % dipotassium hydrogen phosphate, 0.06 w/v % sodium dihydrogen phosphate, 0.05 w/v % magnesium sulfate heptahydrate, 0.5 w/v % calcium carbonate and water, and each flask was sterilized by heating it at 115°C for 30 min, cooled, admixed with 50µg/ml ampicillin, and inoculated with the transformant BRM8 obtained in Experiment 3-2, followed by the incubation at 37°C for 24 hours under rotatory-shaking conditions to obtain a seed culture. To 30-L jar fermenters were added 18 L aliquots of a fresh preparation of the same nutrient culture medium, sterilized similarly as above, admixed with 50 µg/ml ampicillin, and inoculated with 1 v/v % of the seed culture, followed by the incubation at 37°C and a pH of 6-8 for 24 hours under aeration-agitation conditions. The resultant cultures were pooled, treated with ultrasonication to disrupt cells, centrifuged to remove insoluble substances, followed by assaying the enzymatic activity of the resultant supernatant. As a result, one L of the culture contained about 880 units of the recombinant enzyme. The assay of the supernatant conducted by the method in Experiment 1-2 revealed that in this culture was obtained an about 5 ml aqueous solution containing about 160 units/ml of a recombinant enzyme with a specific activity of about 34 units/mg protein.

### Example B-1

### Preparation of trehalose syrup by recombinant enzyme

Potato starch powder was suspended in water to give a concentration of 10 w/w %, and the suspension was adjusted to pH 5.5, admixed with 2 units/g starch of "SPITASE HS", an a-amylase specimen commercialized by Nagase Biochemicals, Ltd., Kyoto, Japan, and heated at 95°C to effect gelatinization and liquefaction. Thereafter, the resultant liquefied solution was autoclaved at 120°C for 20 min to inactivate the remaining enzyme, promptly cooled to 50°C, adjusted to pH 5.0, admixed with 500 units/g starch of an isoamylase specimen commercialized by Hayashibara Biochemical Laboratories, Inc., Okayama, Japan, and 20 units/g starch of a β-amylase specimen commercialized by Nagase Biochemicals, Ltd., Kyoto, Japan, and subjected to an enzymatic reaction at 50°C for 24 hours to obtain a saccharide solution containing about 92 w/w % maltose, d.s.b. The saccharide solution was heated at 100°C for 20 min to inactivate the remaining enzyme, cooled to 10°C, adjusted to pH 7.0, admixed with one unit/g starch of the recombinant enzyme obtained in Example A-1, and subjected to an enzymatic reaction for 96 hours. The reaction mixture was heated at 95°C for 10 min to inactivate the remaining enzyme, cooled, filtered, and, in usual manner, decolored with an activated charcoal, desalted and deionized with an ion-exchange resin, and concentrated to obtain a 70 w/w % syrup in a yield of about 95% to the material starch, d.s.b.

The product contains about 69 w/w % trehalose, d.s.b, and has a relatively-low reducibility because of its DE (dextrose equivalent) 18.2, as well as having a mild sweetness, moderate viscosity and moisture-retaining ability, and these render it arbitrarily useful in a variety of compositions such as food products, cosmetics and pharmaceuticals as a sweetener, taste-improving agent, stabilizer, filler, adjuvant or excipient.

### Example B-2

### Preparation of trehalose powder by recombinant DNA

The reaction mixture obtained in Example B-1 was adjusted to pH 5.0, admixed with 10 units/g starch of "GLUCOZYME", a glucoamylase specimen commercialized by Nagase Biochemicals, Ltd., Kyoto, Japan, and subjected to an enzymatic reaction at 50°C for 24 hours. The reaction mixture thus obtained was heated to inactivate the remaining enzyme, and, in usual manner, decolored, desalted, purified and subjected to ion-exchange column chromatography using "XT-1016 (polymerization degree of 4%)", a cation exchange resin in Na⁺-form commercialized by Tokyo Organic Chemical Industries., Ltd., Tokyo, Japan, to increase the trehalose content. More particularly, the ion-exchange resin, previously suspended in water, was packed in 4 jacketed-stainless steel columns with an inner column diameter of 5.4 cm, and the columns were cascaded in series to give a total column length of 20 m. About 5 v/v % of the reaction mixture was fed to the columns while the inner column temperature was keeping at 60°C, and fractionated by feeding to the columns with 60°C hot water at an SV (space velocity) 0.15, followed by collecting high-trehalose content fractions. The fractions were pooled, and, in usual manner, concentrated, dried *in vacuo*, and pulverized to obtain a trehalose powder in a yield of about 55% to the material, d.s.b.

The product contained about 97 w/w % trehalose, d.s.b, and has a relatively-low reducing power because of its DE (dextrose equivalent) 18.2, as well as having a mild sweetness, moderate viscosity, and moisture-retaining ability, and these render it arbitrarily useful in a variety of compositions such as food products, cosmetics and pharmaceuticals as a sweetener, taste-improving agent, stabilizer, filler, adjuvant or excipient.

### Example B-3

### Preparation of crystalline trehalose powder by recombinant enzyme

A high-trehalose content fraction, obtained by the method in Example B-2, was in usual manner decolored with an activated charcoal, desalted with an ion-exchanger, and concentrated into an about 70 w/w % solution. The concentrate was placed in a crystallizer and gradually cooled while stirring to obtain a massecuite with a crystallization percentage of about 45%. The massecuite was sprayed at a pressure of about 150 kg/cm² from a nozzle equipped at the top of a drying tower while about 85°C hot air was blowing downward from the top of the drying tower, about 45°C hot air was blowing through under a wire-netting conveyer, which was equipped in the basement of the drying tower, to a crystalline powder collected on the conveyer, and the powder was gradually conveying out from the drying tower. Thereafter, the crystalline powder was transferred to an aging tower and aged for 10 hours in the stream of hot air to complete the crystallization and drying. Thus, a hydrous crystalline trehalose powder was obtained in a yield of about 90% to the material, d.s.b.

The product is substantially free from hygroscopicity and readily handleable, and it can be arbitrarily used in a variety compositions such as food products, cosmetics and pharmaceuticals as a sweetener, taste-improving agent, quality-improving agent, stability, filler, adjuvant or excipient.

### Example B-4

### Preparation of anhydrous crystalline trehalose powder by recombinant enzyme

A high-trehalose content fraction, obtained by the method in Example B-2, was purified similarly as in Example B-3, and the resultant solution was transferred to a vessel and boiled under a reduced pressure to obtain a syrup with a moisture content of about 3.0 w/w %. The syrup was placed in a crystallizer, admixed with about one w/w % anhydrous crystalline trehalose as a seed crystal, crystallized at 120°C while stirring, and transferred to a plain aluminum vessel, followed by aging it at 100°C for 6 hours to form a block. The block thus obtained was pulverized with a cutter, dried by fluidized bed drying to obtain an anhydrous crystalline trehalose powder with a moisture content of about 0.3 w/w % in a yield of about 85% to the material, d.s.b.

The product with a strong dehydrating activity can be arbitrarily used as a desiccant for food products, cosmetics and pharmaceuticals, as well as their materials and intermediates, and also used as a white powdery sweetener with a mild sweetness in food products, cosmetics and pharmaceuticals.

As is described above, the present invention is based on the finding of a novel enzyme which forms trehalose or maltose when acts on maltose or trehalose, and it aims to explore a way to produce such an enzyme in an industrial scale and in a considerably-high yield by the recombinant DNA technology. The enzymatic conversion method according to the present invention attains the conversion of maltose into a saccharide composition containing trehalose, glucose and/or maltose in a considerably-high yield. Trehalose has a mild and high-quality sweetness, and does not have a reducing residue within the molecule, and because of these it can readily sweeten food products in general without fear of causing unsatisfactory coloration and deterioration. The recombinant enzyme with a revealed amino acid sequence can be used with a greater safety for the preparation of trehalose which is premised to be used in food products.

Therefore, the present invention is an useful invention which exerts the aforesaid significant action and effect as well as giving a great contribution to this field.

## Claims

1. An isolated DNA which encodes a recombinant enzyme having the following physiochemical properties:
(1) Action
Forming trehalose when acts on maltose, and *vice versa;*
(2) Molecular weight
57,000-67,000 daltons when assayed on sodium dodecyl sulfate polyacrylamide gel electrophoresis (SDS-PAGE); and
(3) Isoelectric point (pI)
4.1-5.1 when assayed on isoelectrophoresis;
and wherein the base sequence of the DNA comprises (i) the base sequence of SEQ ID NO:2, (ii) a complementary base sequence thereto, or (iii) a variant of the base sequence of SEQ ID NO:2 where one or more bases in SEQ ID NO:2 are replaced with other bases by means of the degeneracy of genetic code, or one or more bases of SEQ ID NO:2 are replaced with other bases without altering the inherent activity of the recombinant enzyme.

2. The DNA as claimed in claim 1, which is derived from a microorganism of the genus *Pimelobacter*.

3. A replicable recombinant DNA which contains a self-replicable vector and a DNA as defined in claim 1 or claim 2.

4. The replicable recombinant DNA as claimed in claim 3, wherein said self-replicable vector is plasmid vector Bluescript II SK(+).

5. A transformant which is prepared by introducing the replicable recombinant DNA defined in claim 3 or claim 4 into a host.

6. The transformant as claimed in claim 5, wherein said host is a microorganism of the species *Escherichia coli*.

7. A process for preparing a recombinant enzyme having the following physicochemical properties of:
(1) Action
Forming trehalose when acts on maltose, and *vice versa;*
(2) Molecular weight
57,000-67,000 daltons when assayed on sodium dodecyl sulfate polyacrylamide gel electrophoresis (SDS-PAGE); and
(3) Isoelectric point (pI)
4.1-5.1 when assayed on isoelectrophoresis
and wherein the amino acid sequence of the enzyme comprises either the amino acid sequence of SEQ ID NO:1 or a variant of SEQ ID NO: 1 where (i) one or more amino acids in SEQ ID NO:1 are replaced with other amino acids, (ii) one or more amino acids or SEQ ID NO: I are deleted, or (iii) one or more amino acids are added to SEQ ID NO:1 without altering the inherent activity of the recombinant enzyme, said process comprising:
(a) culturing the transformant as claimed in claim 5 or claim 6 in a nutrient culture medium to form said recombinant enzyme; and
(b) collecting the formed recombinant enzyme from the resultant culture.

8. The process as claimed in claim 7, wherein the collecting technique in the step (b) is one or more members selected from the group consisting of centrifugation, filtration, concentration, salting out, dialysis, separatory sedimentation, ion-exchange chromatography, gel filtration chromatography, hydrophobic chromatography, affinity chromatography, gel electrophoresis and isoelectrophoresis.

9. A process for preparing trehalose, which process comprises a step of producing a recombinant enzyme by the process defined in claim 7 or claim 8, and a step of allowing the recombinant enzyme to act on maltose to form trehalose.

10. The process as claimed in claim 9, wherein the recombinant enzyme acts on maltose contained in a saccharide composition.

11. The process as claimed in claim 9 or claim 10, wherein an effective amount of the recombinant enzyme is allowed to coexist in an aqueous medium containing maltose up to 50 w/v% to effect enzymatic reaction at a temperature of 4-45°C and a pH of 5.5-9.0.

12. The process as claimed in any of claims 9 to 11, which forms a composition containing trehalose up to 80 w/w%, on a dry solid basis.

13. The process as claimed in claim 10, wherein the content of maltose in the saccharide composition is 70 w/w% or more, on a dry solid basis.

## Patentansprüche

1. Isolierte DNA, welche ein rekombinantes Enzym mit den folgenden physikalisch-chemischen Eigenschaften kodiert:
(1) Wirkung
Bildung von Trehalose beim Einwirken auf Maltose und umgekehrt;
(2) Molekulargewicht
57.000 - 67.000 Daltons nach Analyse durch Natriumdodecylsulfat-Polyacrylamid-Gelelektrophorese (SDS-PAGE); und
(3) Isoelektrischer Punkt (pl)
4,1 - 5,1 nach Analyse durch Isoelektrophorese;
und, wobei die Basensequenz der DNA (i) die Basensequenz von SEQ ID NO:2, (ii) eine komplementäre Basensequenz hierzu oder (iii) eine Variante der Basensequenz von SEQ ID NO:2, bei der eine oder mehrere Basen in SEQ ID NO:2 durch andere Basen mittels der Entartung des genetischen Codes ersetzt sind oder eine oder mehrere Basen von SEQ ID NO:2 durch andere Basen ohne Änderung der inhärenten Aktivität des rekombinanten Enzyms ersetzt sind, ist.

2. DNA nach Anspruch 1, welche sich aus einem Mikroorganismus der Gattung *Pimelobacter* ableitet.

3. Replizierbare rekombinante DNA, welche einen selbst-replizierbaren Vektor und eine wie in Anspruch 1 oder Anspruch 2 definierte DNA enthält.

4. Replizierbare rekombinante DNA nach Anspruch 3, wobei der selbstreplizierbare Vektor der Plasmidvektor Blueskript II SK(+) ist.

5. Transformant, welcher durch Einführen der replizierbaren rekombinanten, in Anspruch 3 oder Anspruch 4 definierten DNA in einen Wirt präpariert ist.

6. Transformant nach Anspruch 5, wobei der Wirt ein Mikroorganismus der Art *Escherichia coli* ist.

7. Verfahren zur Herstellung eines rekombinanten Enzyms mit den folgenden physikalisch-chemischen Eigenschaften:
(1) Wirkung
Bildung von Trehalose beim Einwirken auf Maltose und umgekehrt;
(2) Molekulargewicht
57.000 - 67.000 Daltons nach Analyse durch Natriumdodecylsulfat-Polyacrylamid-Gelelektrophorese (SDS-PAGE); und
(3) Isoelektrischer Punkt (pI)
4,1 - 5,1 nach Analyse durch Isoelektrophorese;
und, wobei die Aminosäuresequenz des Enzyms entweder die Aminosäuresequenz von SEQ ID NO:1 oder einer Variante von SEQ ID NO:1, in der ohne Änderung der inhärenten Aktivität des rekombinanten Enzyms (i) eine oder mehrere Aminosäuren in SEQ ID NO:1 durch andere Aminosäuren ersetzt, (ii) eine oder mehrere Aminosäuren in SEQ ID NO:1 deletiert oder (iii) eine oder mehrere Aminosäuren zu SEQ ID NO:1 hinzugefügt sind, enthält, wobei das Verfahren umfasst:
(a) Kultivieren des Transformanten gemäß Anspruch 5 oder Anspruch 6 in einem flüssigen Kulturmedium zwecks Bildung des rekombinanten Enzyms; und
(b) Sammeln des gebildeten rekombinanten Enzyms aus der resultierenden Kultur.

8. Verfahren nach Anspruch 7, wobei die Sammeltechnik in dem Schritt (b) ein oder mehrere Mitglieder ausgewählt aus der aus Zentrifugation, Filtration, Konzentration, Aussalzen, Dialyse, abtrennende Sedimentation, Ionenaustauschchromatographie, Gelfiltrationschromatographie, hydrophobe Chromatographie, Affinitätschromatographie, Gelektrophorese und Isoelektrophorese bestehenden Gruppe sind.

9. Verfahren zur Präparation von Trehalose, welches einen Schritt der Herstellung eines rekombinanten Enzyms durch das in Anspruch 7 oder Anspruch 8 definierte Verfahren und einen Schritt des Erlaubens des rekombinanten Enzyms, auf Maltose einzuwirken, um Trehalose zu bilden, umfasst.

10. Verfahren nach Anspruch 9, wobei das rekombinante Enzym auf in einer Saccharidzusammensetzung enthaltende Maltose einwirkt.

11. Verfahren nach Anspruch 9 oder Anspruch 10, wobei einer effektiven Menge an rekombinanten Enzym erlaubt wird, in einem bis zu 50 Gew.-% Maltose enthaltenden wässrigen Medium zu koexistieren, um eine enzymatische Reaktion bei einer Temperatur von 4-45°C und einem pH von 5,5-9,0 zu bewirken.

12. Verfahren nach einem der Ansprüche 9 bis 11, welches eine Zusammensetzung enthaltend bis zu 80 Gew.-% Trehalose, bezogen auf die trockene Feststoffbasis, bildet.

13. Verfahren nach Anspruch 10, wobei der Gehalt an Maltose in der Saccharidzusammensetzung 70 Gew.-% oder mehr, bezogen auf die trockene Feststoffbasis, ist.

## Revendications

1. ADN isolé qui code pour une enzyme recombinante ayant les propriétés physicochimiques suivantes :
(1) action : formation de tréhalose quand il agit sur le maltose, et inversement ;
(2) masse moléculaire : 57 000-67 000 daltons, la mesure étant effectuée par électrophorèse sur gel de polyacrylamide en présence de dodécylsulfate de sodium (SDS-PAGE) ; et
(3) point isoélectrique (pI) : 4,1-5,1, la mesure utilisant une isoélectrophorèse ;
et où la séquence de base de l'ADN comprend (i) la séquence de base SEQ ID No.2, (ii) une séquence de base qui lui est complémentaire, ou (iii) un variant de la séquence de base SEQ ID No.2, où une ou plusieurs bases de la séquence SEQ ID No.2 sont remplacées par d'autres bases par dégénérescence du code génétique, ou encore une ou plusieurs bases de la séquence SEQ ID No.2 sont remplacées par d'autres bases sans modification de l'activité intrinsèque de l'enzyme recombinante.

2. ADN selon la revendication 1, qui dérive d'un microorganisme du genre *Pimelobacter*.

3. ADN recombinant réplicable, qui contient un vecteur autoréplicable et un ADN selon la revendication 1 ou 2.

4. ADN recombinant réplicable selon la revendication 3, dans lequel ledit vecteur autoréplicable est le vecteur plasmidique Bluescript II SK(+).

5. Transformant qui est préparé par introduction, dans un hôte, de l'ADN recombinant réplicable selon la revendication 3 ou 4.

6. Transformant selon la revendication 5, dans lequel ledit hôte est un microorganisme de l'espèce *Escherichia coli*.

7. Procédé de préparation d'une enzyme recombinante ayant les propriétés physicochimiques suivantes :
(1) action : formation de tréhalose quand il agit sur le maltose, et inversement ;
(2) masse moléculaire : 57 000-67 000 daltons, la mesure étant effectuée par électrophorèse sur gel de polyacrylamide en présence de dodécylsulfate de sodium (SDS-PAGE) ; et
(3) point isoélectrique (pI) : 4,1-5,1, la mesure utilisant une isoélectrophorèse ;
et où la séquence d'acides aminés de l'enzyme comprend la séquence d'acides aminés SEQ ID No.1 ou un variant de la séquence SEQ ID No.1, où (i) un ou plusieurs acides aminés de la séquence SEQ ID No.1 sont remplacés par d'autres acides aminés, (ii) un ou plusieurs acides aminés de la séquence SEQ ID No.1 sont supprimés, ou encore (iii) un ou plusieurs acides aminés sont ajoutés à la séquence SEQ ID No.1 sans modification de l'activité intrinsèque de l'enzyme recombinante, ledit procédé comprenant :
(a) la culture du transformant selon la revendication 5 ou 6 dans un milieu de culture nutritif pour former ladite enzyme recombinante ; et
(b) la collecte de l'enzyme recombinante ainsi formée, à partir de la culture obtenue.

8. Procédé selon la revendication 7, dans lequel la technique de collecte de l'étape (b) est constituée d'un ou plusieurs membres choisis dans le groupe consistant en une centrifugation, une filtration, une concentration, un relargage, une dialyse, une sédimentation par séparation, une chromatographie d'échange d'ions, une chromatographie par filtration sur gel, une chromatographie hydrophobe, une chromatographie d'affinité, une électrophorèse sur gel et une isoélectrophorèse.

9. Procédé de préparation de tréhalose, lequel procédé comprend une étape de production d'une enzyme recombinante par le procédé selon la revendication 7 ou 8, et une étape consistant à permettre à l'enzyme recombinante d'agir sur le maltose pour former du tréhalose.

10. Procédé selon la revendication 9, dans lequel l'enzyme recombinante agit sur le maltose contenu dans une composition de saccharides.

11. Procédé selon la revendication 9 ou 10, dans lequel on laisse une quantité efficace de l'enzyme recombinante coexister dans un milieu aqueux contenant du maltose en une quantité allant jusqu'à 50 % m/v, pour effectuer une réaction enzymatique à une température de 4-45°C et à un pH de 5,5-9,0.

12. Procédé selon l'une quelconque des revendications 9 à 11, qui forme une composition contenant du tréhalose en une quantité allant jusqu'à 80 % m/m, en extrait sec.

13. Procédé selon la revendication 10, dans lequel la teneur de la composition de saccharide en maltose est de 70 % m/m ou plus, en extrait sec.


### SEQUENCE LISTING

(1) GENERAL INFORMATION:
   (i) APPLICANT:
      (A) NAME: KABUSHIKI KAISHA HAYASHIBARA SEIBUTSU KAGAKU KENKYUJO
      (B) STREET: 2-3, 1-chome, Shimoishii, Okayama-shi
      (C) CITY: Okayama
      (E) COUNTRY: Japan
      (F) POSTAL CODE (ZIP): 700
   (ii) TITLE OF INVENTION:RECOMBINANT ENZYME FOR CONVERTING MALTOSE INTO TREHALOSE
   (iii) NUMBER OF SEQUENCES:8
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
   (vi) PRIOR APPLICATION DATA:
      (A) APPLICATION NUMBER:JP 156399/94
      (B) FILING DATE: June 16, 1994
(2) INFORMATION FOR SEQ ID NO:1:
   (i) SEQUENCE CHARACTERISTICS:
      (A) LENGTH:568 amino acids
      (B) TYPE:amino acid
      (D) TOPOLOGY:linear
   (ii) MOLECULE TYPE:peptide
   (xi) SEQUENCE DESCRIPTION:SEQ ID NO:1:
   Ser Thr Val Leu Gly Glu Glu Pro Glu Trp Phe Arg Thr Ala Val Phe
   1               5                   10                  15
   Tyr Glu Val Leu Val Arg Ser Phe Arg Asp Pro Asn Ala Gly Gly Thr
               20                  25                  30
   Gly Asp Phe Arg Gly Leu Ala Glu Lys Leu Asp Tyr Leu Gln Trp Leu
           35                  40                  45
   Gly Val Asp Cys Leu Trp Val Pro Pro Phe Phe Ser Ser Pro Leu Arg
       50                  55                  60
   Asp Gly Gly Tyr Asp Val Ala Asp Tyr Thr Gly Ile Leu Pro Glu Ile
   65                  70                  75                  80
   Gly Thr Val Glu Asp Phe His Ala Phe Leu Asp Gly Ala His Glu Arg
                   85                  90                  95
   Gly Ile Arg Val Ile Ile Asp Phe Val Met Asn His Thr Ser Asp Ala
               100                 105                 110
   His Pro Trp Phe Gln Ala Ser Arg Ser Asp Pro Asp Gly Pro Tyr Gly
           115                 120                 125
   Asp Phe Tyr Val Trp Ser Asp Thr Asp Glu Leu Tyr Gln Asp Ala Arg
       130                 135                 140
   Val Ile Phe Val Asp Thr Glu Pro Ser Asn Trp Thr Trp Asp Gln Thr
   145                 150                 155                 160
   Arg Gly Gln Tyr Tyr Trp His Arg Phe Phe His His Gln Pro Asp Leu
                   165                 170                 175
   Asn Phe Asp Asn Pro Lys Val Gln Asp Ala Met Leu Glu Ala Met Ala
               180                 185                 190
   Phe Trp Leu Asp Met Gly Leu Asp Gly Phe Arg Leu Asp Ala Val Pro
           195                 200                 205
   Tyr Leu Tyr Glu Arg Pro Gly Thr Asn Gly Glu Asn Leu Pro Glu Thr
       210                 215                 220
   His Glu Met Leu Lys Arg Val Arg Arg Phe Val Asp Asp Asn Tyr Pro
   225                 230                 235                 240
   Asp Arg Val Leu Leu Tyr Glu Ala Asn Gln Trp Pro Thr Asp Val Val
                       245                 250                 255
   Glu Tyr Phe Gly Pro Glu Glu Arg Glu Asp Gly Thr Val Val Gly Pro
                   260                 265                 270
   Glu Ser His Met Ala Phe His Phe Pro Val Met Pro Arg Ile Phe Met
               275                 280                 285
   Ala Val Arg Arg Glu Ser Arg Phe Pro Ile Ser Glu Ile Met Glu Gln
           290                 295                 300
   Thr Pro Ala Ile Pro Glu Gly Cys Gln Trp Gly Ile Phe Leu Arg Asn
   305                 310                 315                 320
   His Asp Glu Leu Thr Leu Glu Met Val Thr Asp Glu Asp Arg Asp Tyr
                       325                 330                 335
   Met Trp Gly Glu Tyr Ala Lys Asp Pro Arg Met Lys Ala Asn Ile Gly
                   340                 345                 350
   Ile Arg Arg Arg Leu Ala Pro Leu Leu Asp Asn Asp Thr Asn Gln Ile
               355                 360                 365
   Glu Leu Phe Thr Ala Leu Leu Leu Ser Leu Pro Gly Ser Pro Val Leu
           370                 375                 380
   Tyr Tyr Gly Asp Glu Ile Gly Met Gly Asp Asn Ile Trp Leu Gly Asp
   385                 390                 395                 400
   Arg Asp Gly Val Arg Thr Pro Met Gln Arg Thr Pro Asp Arg Asn Val
                       405                 410                 415
   Gly Phe Ser Ala Ala Thr Pro Gly Lys Leu His Leu Pro Thr Ile Gln
                   420                 425                 430
   Asp Pro Val Tyr Gly Tyr Gln Ser Val Asn Val Glu Ala Gln Leu Glu
               435                 440                 445
   Asn Pro Ser Ser Leu Leu His Trp Thr Arg Arg Met Ile His Ile Arg
           450                 455                 460
   Arg Gln Arg Asp Ala Phe Gly Leu Gly Thr Phe Glu Asp Leu Gly Gly
   465                 470                 475                 480
   Ser Asn Pro Ala Val Leu Ser Tyr Val Arg Glu Leu Pro Gly Asp Gly
                       485                 490                 495
   Gly Asp Asp Val Ile Leu Cys Val Asn Asn Leu Ser Arg Phe Pro Gln
                   500                 505                 510
   Pro Val Glu Leu Asp Leu Arg Lys Tyr Glu Gly Arg Val Pro Val Glu
               515                 520                 525
   Leu Ile Gly Gly Val Pro Phe Pro Ala Val Gly Glu Leu Pro Tyr Leu
           530                 535                 540
   Leu Thr Leu Ser Gly His Gly Phe Tyr Trp Phe Arg Leu Thr Asp Pro
   545                 550                 555                 560
   Asp Thr Thr Gly Arg Pro Val Leu
                       565
(3) INFORMATION FOR SEQ ID NO:2:
   (i) SEQUENCE CHARACTERISTICS:
      (A) LENGTH:1704 base pairs
      (B) TYPE:nucleic acid
      (D) TOPOLOGY:linear
   (xi) SEQUENCE DESCRIPTION:SEQ ID NO:2:
   TCGACCGTCC TGGGCGAGGA ACCCGAGTGG TTCCGCACGG CGGTCTTCTA CGAGGTCCTG   60
   GTGCGGTCCT TCCGGGACCC CAACGCCGGC GGCACGGGTG ACTTCCGCGG CCTGGCGGAG  120
   AAGCTCGACT ACCTGCAGTG GCTCGGCGTC GACTGCCTGT GGGTGCCGCC GTTCTTCAGC  180
   TCGCCGCTGC GCGACGGCGG GTACGACGTC GCCGACTACA CCGGGATCCT CCCGGAGATC  240
   GGCACGGTCG AGGACTTCCA CGCCTTCCTC GACGGCGCGC ACGAGCGCGG GATCCGGGTG  300
   ATCATCGACT TCGTCATGAA CCACACGAGT GACGCGCACC CGTGGTTCCA GGCCTCCCGC  360
   AGCGATCCCG ACGGCCCGTA CGGCGACTTC TACGTCTGGT CCGACACCGA CGAGCTCTAC  420
   CAGGACGCGC GGGTGATCTT CGTCGACACC GAGCCGTCGA ACTGGACGTG GGACCAGACC  480
   CGCGGCCAGT ACTACTGGCA CCGCTTCTTC CACCACCAGC CCGACCTGAA CTTCGACAAC  540
   CCGAAGGTCC AGGACGCCAT GCTGGAGGCG ATGGCGTTCT GGCTCGACAT GGGCCTCGAC  600
   GGCTTCCGGC TCGACGCGGT GCCCTACCTC TACGAGCGTC CCGGCACCAA CGGCGAGAAC  660
   CTCCCCGAGA CGCACGAGAT GCTCAAGCGG GTGCGGCGCT TCGTCGACGA CAACTACCCC  720
   GACCGGGTGC TGCTGTACGA GGCGAACCAG TGGCCGACCG ACGTGGTGGA GTACTTCGGG  780
   CCCGAGGAGC GTGAGGACGG CACGGTCGTC GGGCCCGAGA GTCACATGGC CTTCCACTTC  840
   CCGGTGATGC CGCGCATCTT CATGGCGGTG CGCCGCGAGT CGCGCTTCCC GATCTCGGAG  900
   ATCATGGAGC AGACGCCGGC GATCCCGGAG GGCTGCCAGT GGGGCATCTT CCTGCGCAAC  960
   CACGACGAGC TGACCCTCGA GATGGTCACC GACGAGGACC GCGACTACAT GTGGGGCGAG 1020
   TACGCCAAGG ACCCCCGCAT GAAGGCCAAC ATCGGCATCC GGCGGCGGCT CGCGCCGCTG 1080
   CTCGACAACG ACACGAACCA GATCGAGCTG TTCACCGCGC TGCTGCTGTC GCTGCCCGGC 1140
   TCCCCCGTCC TGTACTACGG CGACGAGATC GGCATGGGCG ACAACATCTG GCTCGGTGAC 1200
   CGCGACGGCG TGCGTACGCC GATGCAGCGG ACCCCCGACC GCAACGTCGG CTTCTCGGCG 1260
   GCCACGCCCG GCAAGCTGCA CCTGCCGACG ATCCAGGACC CGGTCTACGG CTACCAGAGC 1320
   GTCAACGTCG AGGCGCAGCT GGAGAACCCC TCCTCGCTGC TGCACTGGAC CCGCCGGATG 1380
   ATCCACATCC GCCGCCAGCG CGACGCCTTC GGGCTGGGCA CCTTCGAGGA CCTCGGCGGC 1440
   TCGAACCCGG CGGTGCTGTC CTACGTGCGC GAGCTGCCGG GCGACGGGGG CGACGACGTG 1500
   ATCCTCTGCG TCAACAACCT GTCCCGCTTC CCGCAGCCGG TCGAGCTCGA CCTCCGGAAG 1560
   TACGAGGGCC GGGTACCGGT GGAGCTGATC GGCGGCGTGC CGTTCCCCGC CGTCGGGGAG 1620
   CTCCCGTATC TCCTGACGCT CAGCGGGCAC GGCTTCTACT GGTTCCGGCT CACGGATCCG 1680
   GACACGACCG GGAGGCCCGT CCTG                                       1704
(4) INFORMATION FOR SEQ ID NO:3:
   (i) SEQUENCE CHARACTERISTICS:
      (A) LENGTH:20 amino acids
      (B) TYPE:amino acid
      (D) TOPOLOGY:linear
   (ii) MOLECULE TYPE:peptide
   (xi) SEQUENCE DESCRIPTION:SEQ ID NO:3:
   Ser Thr Val Leu Gly Glu Glu Pro Glu Trp Phe Arg Thr Ala Val Phe Tyr
   1               5                   10                  15
   Glu Val Leu
           20
(5) INFORMATION FOR SEQ ID NO:4:
   (i) SEQUENCE CHARACTERISTICS:
      (A) LENGTH:11 amino acids
      (B) TYPE:amino acid
      (D) TOPOLOGY:linear
   (ii) MOLECULE TYPE:peptide
   (xi) SEQUENCE DESCRIPTION:SEQ ID NO:4:
   Val Gln Asp Ala Met Leu Glu Ala Met Ala Phe
   1               5                   10
(6) INFORMATION FOR SEQ ID NO:5:
   (i) SEQUENCE CHARACTERISTICS:
      (A) LENGTH:1704 base pairs
      (B) TYPE:nucleic acid
      (D) TOPOLOGY:linear
   (ii) MOLECULE TYPE:genomic DNA
   (vi) ORIGINAL SOURCE:
      (A) ORGANISM:Pimelobacter sp.
      (B) INDIVIDUAL ISOLATE:R48 (FERM BP-4315)
   (xi) SEQUENCE DESCRIPTION:SEQ ID NO:5:
(7) INFORMATION FOR SEQ ID NO:6:
   (i) SEQUENCE CHARACTERISTICS:
      (A) LENGTH:17 base pairs
      (B) TYPE:nucleic acid
      (D) TOPOLOGY:linear
   (ii) MOLECULE TYPE:probe
   (xi) SEQUENCE DESCRIPTION:SEQ ID NO:6:
   GARGARCCNG ARTGGTT                         17
(8) INFORMATION FOR SEQ ID NO:7:
   (i) SEQUENCE CHARACTERISTICS:
      (A) LENGTH:17 base pairs
      (B) TYPE:nucleic acid
      (D) TOPOLOGY:linear
   (ii) MOLECULE TYPE:probe
   (xi) SEQUENCE DESCRIPTION:SEQ ID NO:7:
   ATGYTNGARG CNATGGC                         17
(9) INFORMATION FOR SEQ ID NO:8:
   (i) SEQUENCE CHARACTERISTICS:
      (A) LENGTH:17 base pairs
      (B) TYPE:nucleic acid
      (D) TOPOLOGY:linear
   (ii) MOLECULE TYPE:primer
   (xi) SEQUENCE DESCRIPTION:SEQ ID NO:8:
   GTAAAACGAC GGCCAGT                         17